# EUROPEAN PATENT SPECIFICATION

(11) **EP 3 834 821 B1**
(45) Date of publication and mention of the grant of the patent: **24.12.2025**
(21) Application number: 19871224.2
(22) Date of filing: 29.09.2019
(51) Int. Cl.: A61K 9/20, A61K 31/365, A61K 47/38, A61K 47/36

(54) **DROPPING PILL COMPRISING GINKGO TERPENE LACTONES AS ACTIVE COMPONENTS AND PREPARATION METHOD THEREFOR**
TROPFPILLE MIT GINKGO-TERPEN-LACTONEN ALS WIRKSTOFFE UND VERFAHREN ZU IHRER HERSTELLUNG
PILULE À AVALER COMPRENANT DES TERPÈNE LACTONES DU GINKGO UTILISÉES COMME CONSTITUANTS ACTIFS ET SON PROCÉDÉ DE PRÉPARATION

(30) Priority: 08.10.2018 CN 201811165409
(43) Date of publication of application: 16.06.2021
(73) Proprietor: Chengdu Baiyu Pharmaceutical Co., Ltd., Chengdu, Sichuan 611130 (CN)
(72) Inventor: CHEN, Yan, Chengdu, Sichuan 611130 (CN); YANG, Yong, Chengdu, Sichuan 611130 (CN)
(74) Representative: Novagraaf Group
(86) International application number: PCT/CN2019/108918
(87) International publication number: WO 2020/073844

(56) References cited:
- CN-A- 1 634 039
- CN-A- 101 317 872
- CN-A- 101 444 545
- CN-A- 106 924 197
- CN-A- 107 753 445
- CN-B- 101 485 697

## Description

### CROSS REFERENCE

The present application claims priority to Chinese Patent Application No. 201811165409.3, filed on October 8, 2018 and entitled "dropping pill comprising ginkgo terpene lactones as active components and preparation method therefor".

### TECHNICAL FIELD

The present application belongs to the field of pharmaceutical preparations, and in particular relates to a dropping pill comprising an active component consisting essentially of ginkgo terpene lactone and a preparation method therefor.

### BACKGROUND

Ginkgo terpene lactones are terpenoid compounds, mainly including ginkgolide A, ginkgolide B, ginkgolide C, ginkgolide M, ginkgolide J and bilobalide, chemical structures of which are as follows: 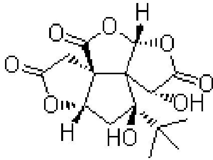 bilobalide, 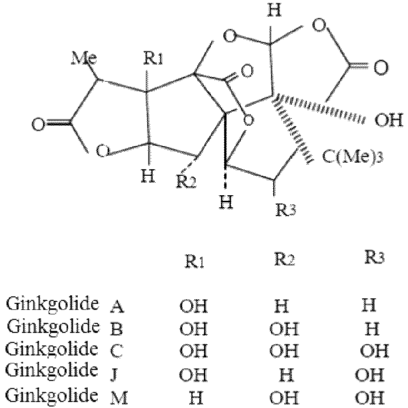 ginkgolide. Pharmacological research results show that ginkgo terpene lactones are natural human PAF receptor antagonist, potent and selective antagonists against glycine receptors (GlyRs), and peripheral benzodiazepine receptor antagonists. Therefore, ginkgo terpene lactones may be effective in treating the above-mentioned receptors-related diseases, and have pharmacological effects such as expanding coronary blood vessels, improving cerebral circulation, inhibiting platelet aggregation, preventing atherosclerosis, antagonizing vascular permeability increase, and relieving bronchoconstriction. Ginkgo terpene lactones have protective effects against heart and brain ischemia damages and provide protection for central nervous system, have anti-aging effect, anti-shock effect, anti-allergic effect, anti-bacterial effect, antiinflammatory effect, anti-tumor effect, anti-rejection effect in organ transplantation, etc., and also have certain effects against liver and kidney damages.

In summary, ginkgo terpene lactones have broad clinical application prospects. However, ginkgo terpene lactones are poorly water-soluble drugs with low bioavailability for absorption in the body, which brings certain difficulties to drug development. Most of the existing ginkgo terpene lactone preparations are oral preparations and injections, wherein ordinary oral preparations have limited bioavailability, affecting clinical efficacy of drugs. A dropping pill of ginkgo terpene lactone disclosed in recent years partially solves the problem of drug solubility, but there is still a technical problem that the dissolution rate is limited. For example, Chinese patent CN 101422456B actually discloses a preparation method of ginkgolide dropping pill and quality detection method thereof, wherein the technical problems of the preparation of ginkgolide dropping pill and the identification and content determination of active components are solved. However, the problem of drug dissolution rate is not mentioned in its disclosure. For another example, Chinese Patent application CN 106924197A discloses a ginkgolide dropping pill and a preparation method thereof, wherein the aging phenomenon of the dropping pill preparation is solved by optimizing the formulation of the dropping pill preparation, but there are still technical problems of limited dissolution rate of active components and low bioavailability. Chinese Patent application CN101444545A relates to a Ginkgo leaf drop pill and a method of manufacture thereof. It discloses the preparation of Ginkgo leaf extract into drop pills, which is used for heart and cerebrovascular diseases. Chinese patent CN101485697B discloses a ginkgo ester dispersible tablet. Ginkgo ester refers to flavonoids and lactones. Chinese Patent application CN107753445A discloses a ginkgolide K dropping pill, comprising ginkgolide K, water-soluble matrix and a solubilizer. It can be seen that the above patents or patent applications do not address the problem of limited dissolution rate of dropping pill of ginkgo terpene lactone.

Furthermore, the formulation of the dropping pill preparation is unique, so there is a need to study the compatibility of its excipients and the preparation method based on the inherent property of ginkgo terpene lactones.

### SUMMARY

In order to solve the above technical problems, the present application provides a dropping pill comprising an active component consisting essentially of ginkgo terpene lactone and a preparation method therefor. The dropping pill can be dissolved quickly and has good bioavailability.

The present application adopts the following technical solutions to solve the above technical problems.

In one aspect, the present application provides a dropping pill comprising i) an active component; ii) a filler having a rigid molecular structure, and/or a filler having a loose and porous surface; and iii) a matrix, wherein the active component consists essentially of ginkgo terpene lactone,
the filler having a rigid molecular structure is selected from the group consisting of microcrystalline cellulose, methyl cellulose, ethyl cellulose, calcium hydrogen phosphate, calcium dihydrogen phosphate, calcium carbonate, calcium sulfate dihydrate, lactose, mannitol, cellulose lactose, and any combination thereof,
the filler having a loose and porous surface is selected from the group consisting of lactose, mannitol, cellulose lactose, any combination thereof, and
the dropping pill comprises 5-15 parts by weight of the ginkgo terpene lactone, 20-40 parts by weight of a matrix, and 1-20 parts by weight of the filler.

The ginkgo terpene lactone of the present application may be in the form of an extract of ginkgo biloba, or a monomer compound with high purity or a composition comprising the same.

Preferably, the microcrystalline cellulose has a bulk density of 0.21 g/cm³ to 0.42 g/cm³.

In a specific embodiment, the microcrystalline cellulose is one or more selected from silicified microcrystalline cellulose SMCC 50, microcrystalline cellulose UF711, and microcrystalline cellulose PH302.

Preferably, the lactose and mannitol are prepared by spray drying.

The filler is a filler having a rigid molecular structure and/or a filler having a loose and porous surface as defined above.

The matrix is preferably PEG, polyvinylpyrrolidone, polyoxyethylene stearate, poloxamer, hydrogenated vegetable oil, insect wax, glyceryl monostearate or/and gelatin.

The dropping pill of the present application further comprises 0.1-4 parts by weight of a surfactant.

Preferably, the surfactant is selected from the group consisting of polysorbate-80, lecithin and carbomer.

More preferably, the surfactant is polysorbate-80.

The dropping pill of the present application further comprises 0.1-2.5 parts by weight of a disintegrant.

Preferably, the disintegrant is selected from the group consisting of sodium carboxymethyl cellulose, dry starch, low-substituted hydroxypropyl cellulose, cross-linked polyvinylpyrrolidone and cross-linked sodium carboxymethyl cellulose.

More preferably, the disintegrant is sodium carboxymethyl cellulose.

Preferably, the filler is a mixture of microcrystalline cellulose and lactose.

Preferably, a weight ratio of the microcrystalline cellulose to lactose is (4-1): (1-4).

In the second aspect, the present invention provides a method for preparing the dropping pill, wherein the method comprises the following steps of:
a) co-crushing or mixing the ginkgo terpene lactone and the filler to obtain a fine powder;
b) mixing a matrix with other components and heating them to melt, adding the fine powder obtained in step a) thereto under stirring to obtain a mixture; and
c) dripping the mixture obtained in step b) with a dripper, and cooling with a condensate to form a dripping pill.

Preferably, the crushing in step a) is crushing by gas impact and/or crushing by mechanical shear.

More preferably, the crushing by gas impact is performed at a pressure of 0.5-0.8 MPa.

In the present application, the ginkgo terpene lactone and the filler are crushed together, which is more conducive to the rapid dissolution of the ginkgo terpene lactone. Compared with the prior art, the present application has following advantages: Ginkgo terpene lactone as active components of the dropping pill disclosed in the present application is dissolved quickly. Animal test results show that ginkgo terpene lactone as active components of the dropping pill disclosed in the present application can enter the blood quickly, thereby showing effect more quickly and achieving a therapeutic effect.

### DESCRIPTION OF DRAWINGS

Figure 1 shows a dissolution curve of ginkgo terpene lactone (pH 1.0);
Figure 2 shows a dissolution curve of ginkgo terpene lactone (pH 2.5);
Figure 3 shows a drug concentration-time curve of ginkgo terpene lactone (in rabbits).

### DETAILED DESCRIPTION OF THE EMBODIMENTS

Unless otherwise specified, raw materials and equipment mentioned in the embodiments of the present application are all known products and are commercially available.

By selecting fillers and other auxiliary materials, the dropping pill of the present application solves the problems in the prior art. The dissolution rate of ginkgo terpene lactone is significantly increased. Subsequent animal experiments have further confirmed that the dropping pill prepared with the above ginkgo terpene lactone can reach a maximum concentration in blood faster than dropping pills of ginkgo terpene lactone prepared by the conventional formulation and method, that is, Tmax appears earlier. Inventors observed that dropping pills comprising the ginkgo terpene lactone as an active component and one or more other active components can also have the above beneficial effects.

In order to simply and directly show the above beneficial effects, formulations, dissolution curves and concentration-time curves are given only for the dropping pill comprising a ginkgo terpene lactone as an active component in the embodiments of the present application. It should not be understood that the active components of the dropping pill of the present application only comprises ginkgo terpene lactone. It should be understood that the active components of the dropping pill of the present application at least contain ginkgo terpene lactone.

The ginkgo terpene lactone of the present application can be an extract from ginkgo biloba, or a monomer compound with high purity or a composition comprising the same.

The ginkgo terpene lactone of the present application can be separated and purified by the existing technology, especially a solvent extraction method, a column extraction method, a solvent extraction-column extraction method, a supercritical extraction method, and a chromatography or column chromatography purification method. Preferably, the ginkgo terpene lactone of the present application is obtained by the method disclosed in WO2013159412A1.

The ginkgo terpene lactone of the present application can also be available commercially.

Fillers, also known as diluents, are mainly used to increase the weight and volume of tablets in the prior art, and are auxiliary materials that facilitate molding and dose dividing. Common fillers for solid preparations include starch, compressible starch, powdered sugar, lactose, microcrystalline cellulose, inorganic salts, absorbents, and sugar alcohols, etc.

As used in the present application, the term "bulk density", also called "packing density", refers to the weight per unit volume in micromeritics.

The filler in the present application refers to a filler having a rigid molecular structure, and is selected from a group consisting of microcrystalline cellulose, methyl cellulose, ethyl cellulose, calcium hydrogen phosphate, calcium dihydrogen phosphate, calcium carbonate, calcium sulfate dihydrate, lactose, mannitol, cellulose lactose. Specifically, the microcrystalline cellulose has a bulk density of 0.21 g/cm³ to 0.42 g/cm³. The microcrystalline cellulose is preferably one or more of silicified microcrystalline cellulose SMCC 50, microcrystalline cellulose UF711, and microcrystalline cellulose PH302.

The filler of the present application further refers to a filler having a loose and porous surface, and is selected from a group consisting of lactose, mannitol, and cellulosic lactose, which are commercially available or prepared by the existing technology, such as lactose and mannitol prepared by spray drying method. Ginkgo terpene lactone itself is difficult to be dissolved in water. By dispersing it on the loose and porous surface of the filler, when the pharmaceutical preparation contacts with for example a digestive juice, the filler is dissolved quickly, leaving behind highly dispersed ginkgo terpene lactone, thereby achieving the beneficial effect of increasing the dissolution rate of ginkgo terpene lactone.

It is found in the present application that when a mixture of microcrystalline cellulose and lactose in a weight ratio of (4-1): (1-4) is used as a filler, ginkgolide shows the fastest in vitro dissolution rate and the smallest Tmax value.

Examples of the microcrystalline cellulose having a specific bulk density include UF711, UF702, and PH series purchased from Asahi Kasei Chemicals Corporation, and silicified microcrystalline cellulose SMCC 50 purchased from J. RETTENMAIER & SÖHNE GROUP. In some embodiments, the filler of the present application can be a filler having a rigid molecular structure such as microcrystalline cellulose UF711 (having a bulk density of 0.22 g/cm³) (Example 7), microcrystalline cellulose UF702 (having a bulk density of 0.29 g/cm³) (formulation 2 ), microcrystalline cellulose PH101 (having a bulk density of 0.29 g/cm³) (formulation 3), microcrystalline cellulose PH103 (bulk density 0.31 g/cm3) (formulation 4), microcrystalline cellulose PH-F20JP (having a bulk density of 0.23 g/cm³) (formulation 5), microcrystalline cellulose PH302 (having a bulk density of 0.42 g/cm³) (Example 1), silicified microcrystalline cellulose SMCC 50 (having a bulk density of 025-0.37 g/cm³) (Example 5), microcrystalline cellulose KG802 (having a bulk density of 0.21 g/cm³) (formulation 3), can also be a filler having a loose and porous surface such as lactose (Example 2, Example 8, etc.), mannitol (example 6), inorganic salts having a rigid molecular structure such as calcium hydrogen phosphate (Example 4), calcium dihydrogen phosphate and calcium carbonate (formulation 4), and calcium sulfate dihydrate (formulation 5), and can be other fillers having a rigid molecular structure and a loose and porous surface such as cellulose lactose (formulations 1 and 2). In a specific embodiment, the filler may also be a combination of one or more of the above fillers (such as Example 7, Example 8, and Example 9).

The dropping pill of the present application may also comprise a matrix, such as PEG, polyvinylpyrrolidone (PVP), polyoxyethylene stearate, poloxamer, hydrogenated vegetable oil, insect wax, glyceryl monostearate or gelatin, etc..

The dropping pill of the present application may also comprise a surfactant, such as polysorbate-80, lecithin or carbomer.

The dropping pill of the present invention may also comprise a disintegrant, such as sodium carboxymethyl cellulose (CMC-Na), dry starch, low-substituted hydroxypropyl cellulose (L-HPC), cross-linked polyvinylpyrrolidone (cross-linked PVP), and croscarmellose sodium (CCNa).

In the method for preparing the dropping pill comprising a ginkgo terpene lactone as an active component, the condensate used can be an aqueous condensate, such as water, ethanol of different concentrations, or an oily condensate, such as liquid paraffin, simethicone, vegetable oil, gasoline or mixtures thereof.

Examples of formulations of dropping pill that have technical effects (in vitro dissolution rate or Tmax value) equivalent to or close to those of Examples 1-9 are listed as follows. Microcrystalline cellulose such as UF711, UF702, PH series purchased from Asahi Kasei Chemicals Corporation are used as examples.

### Formulation 1

| | |
|---|---|
| ginkgo terpene lactone | 1kg |
| Cellulose lactose | 1kg |
| polyethylene glycol 4000 | 3kg |
| polysorbate-80 | 0.01kg |
| dry starch | 0.01kg |
| prepared into | 100,000 dripping pills |

### Formulation 2

| | |
|---|---|
| ginkgo terpene lactone | 1kg |
| Cellulose lactose | 0.05kg |
| microcrystalline cellulose UF702 | 0.05kg |
| polyethylene glycol 4000 | 3kg |
| polysorbate-80 | 0.2kg |
| prepared into | 100,000 dripping pills |

### Formulation 3

| | |
|---|---|
| ginkgo terpene lactone | 1kg |
| microcrystalline cellulose PH101 | 0.5kg |
| microcrystalline cellulose KG802 | 0.5kg |
| calcium hydrogen phosphate | 0.1kg |
| polyethylene glycol 4000 | 3kg |
| polysorbate-80 | 0.4kg |
| prepared into | 100,000 dripping pills |

### Formulation 4

| | |
|---|---|
| ginkgo terpene lactone | 1kg |
| lactose | 0.7kg |
| microcrystalline cellulose PH103 | 0.2kg |
| calcium dihydrogen phosphate | 0.1kg |
| calcium carbonate | 0.1kg |
| polyethylene glycol 6000 | 3kg |
| polysorbate-80 | 0.2kg |
| prepared into | 100,000 dripping pills |

### Formulation 5

| | |
|---|---|
| ginkgo terpene lactone | 1kg |
| calcium sulfate dihydrate | 0.6kg |
| microcrystalline cellulose PH-F20JP | 1.4kg |
| polyethylene glycol 4000 | 3kg |
| polysorbate-80 | 0.2kg |
| low-substituted hydroxypropyl cellulose | 0.25kg |
| prepared into | 100,000 dripping pills |

### Formulation 6

| | |
|---|---|
| ginkgo terpene lactone | 1kg |
| calcium dihydrogen phosphate | 0.5kg |
| polyethylene glycol 4000 | 3kg |
| polysorbate-80 | 0.2kg |
| cross-linked sodium carboxymethyl cellulose | 0.1kg |
| prepared into | 100,000 dripping pills |

### Formulation 7

| | |
|---|---|
| ginkgo terpene lactone | 1kg |
| mannitol | 2kg |
| polyethylene glycol 4000 | 2.8kg |
| polyethylene glycol 6000 | 1.2kg |
| cross-linked polyvinylpyrrolidone | 0.01kg |
| prepared into | 100,000 dripping pills |

### Formulation 8

| | |
|---|---|
| ginkgo terpene lactone | 1kg |
| lactose | 0.5kg |
| Cellulose lactose | 0.7kg |
| polyethylene glycol 4000 | 2.0kg |
| cross-linked sodium carboxymethyl cellulose | 0.05kg |
| prepared into | 100,000 dripping pills |

Hereinafter, the above-mentioned content of the present application will be further described through preferred embodiments. However, it should not be understood that the scope of the present application is limited to the following specific embodiments.

Unless otherwise specified, the raw materials and equipment used in the following examples are all known products and commercially available. The microcrystalline cellulose PH-302 having a bulk density of 0.42 g/cm³ and UF711 having a bulk density of 0.22 g/cm³ in the following Examples 1-9 were purchased from Asahi Kasei Chemicals Corporation, and silicified microcrystalline cellulose is product SMCC 50 having a bulk density of 0.25-0.37 g/cm³ purchased from JRS (J. RETTENMAIER & SÖHNE GROUP).

### Example 1

Formulation:

| | |
|---|---|
| ginkgo terpene lactone | 1kg |
| microcrystalline cellulose PH302 | 1kg |
| polyethylene glycol 4000 | 3kg |
| polysorbate-80 | 0.2kg |
| prepared into | 100,000 dripping pills |

Preparation process: the ginkgo terpene lactone was passed through a 60-mesh sieve, and other auxiliary materials were passed through a 100-mesh sieve. 1 kg of the ginkgo terpene lactone was mixed with 1 kg of microcrystalline cellulose PH302 in a tank mixer for 10 minutes to obtain a mixture. The mixture was crushed in a jet mill at an air pressure of 0.5 MPa to obtain a fine powder with d90 less than 20µm. 3kg polyethylene glycol 4000 and 0.2kg polysorbate-80 were heated to melt at 80°C, and then the fine powder was slowly added thereto under stirring to obtain a second mixture. The second mixture was dripped with a 2.4mm dripper, and then cooled and molded in liquid paraffin at 15°C to form 100,000 dropping pills, with each pill 52 mg.

In the preparation process of example 1, ginkgo terpene lactone and the filler microcrystalline cellulose were crushed together, so that the particle size of the raw materials was reduced by the crushing, which is beneficial to the rapid dissolution of ginkgo terpene lactone.

### Example 2

Formulation:

| | |
|---|---|
| ginkgo terpene lactone | 1kg |
| lactose | 1kg |
| polyethylene glycol 6000 | 3kg |
| polysorbate-80 | 0.2kg |
| prepared into | 100,000 dripping pills |

The lactose used in this formulation was prepared by spray drying.

Preparation process: the ginkgo terpene lactone was passed through a 60-mesh sieve, and other auxiliary materials were passed through a 100-mesh sieve. The ginkgo terpene lactone was crushed in a jet mill at an air pressure of 0.5 MPa to obtain a fine powder with d90 less than 50µm. 1 kg of the fine powder of ginkgo terpene lactone was mixed thoroughly with 1kg lactose, so that they were fully contact with each other to obtain a first mixture. 3kg polyethylene glycol 6000 and 0.2kg polysorbate-80 were heated to melt at 80°C, and then the first mixture was slowly added thereto under stirring to obtain a second mixture. The second mixture was dripped with a 2.4mm dripper, and then cooled and molded in liquid paraffin at 15°C to form 100,000 dropping pills, with each pill 52 mg.

### Example 3

Formulation:

| | |
|---|---|
| ginkgo terpene lactone | 0.5kg |
| methylcellulose | 1kg |
| polyethylene glycol 400 | 0.2kg |
| polyethylene glycol 4000 | 3kg |
| polysorbate-80 | 0.2kg |
| prepared into | 50,000 dripping pills |

Preparation process: the ginkgo terpene lactone was passed through a 60-mesh sieve, and other auxiliary materials were passed through a 100-mesh sieve. 0.5 kg of the ginkgo terpene lactone was mixed with 1 kg of methylcellulose in a tank mixer for 10 minutes to obtain a first mixture. The first mixture was crushed in a jet mill at an air pressure of 0.6 MPa to obtain a fine powder with d90 less than 20µm. The fine powder was mixed thoroughly with 0.2 kg of polyethylene glycol 400, so that they were fully contact with each other to obtain a second mixture. 3kg polyethylene glycol 4000 and 0.2kg polysorbate-80 were heated to melt at 80°C, and then the second mixture was slowly added thereto under stirring to obtain a third mixture. The third mixture was dripped with a 2.4mm dripper, and then cooled and molded in simethicone 100# at 15°C to form 50,000 dropping pills, with each pill 98 mg.

### Example 4

Formulation:

| | |
|---|---|
| ginkgo terpene lactone | 15kg |
| calcium hydrogen phosphate | 0.6kg |
| polyethylene glycol 4000 | 3kg |
| polysorbate-80 | 0.2kg |
| prepared into | 150,000 dripping pills |

Preparation process: the ginkgo terpene lactone was passed through a 60-mesh sieve, and other auxiliary materials were passed through a 100-mesh sieve. 15 kg of the ginkgo terpene lactone was mixed with 0.6 kg of calcium hydrogen phosphate in a tank mixer for 10 minutes to obtain a mixture. The mixture was crushed in a jet mill at an air pressure of 0.8 MPa to obtain a fine powder with d90 less than 20µm. The fine powder was mixed thoroughly with 0.2 kg of polysorbate-80, so that they were contact with each other to obtain a second mixture. 3kg polyethylene glycol 4000 was heated to be melt at 80°C, and then the second mixture was slowly added thereto under stirring to obtain a third mixture. The third mixture was dripped with a 2.2mm dripper, and then cooled and molded in simethicone 100# at 15°C to form 150,000 dropping pills, with each pill 125.3 mg.

### Example 5

Formulation:

| | |
|---|---|
| ginkgo terpene lactone | 1kg |
| silicified microcrystalline celluloseSMCC 50 | 0.5kg |
| methylcellulose | 0.1kg |
| polyethylene glycol 400 | 0.2kg |
| polyethylene glycol 4000 | 3kg |
| hydroxypropyl methylcellulose K4 | 0.1kg |
| polysorbate-80 | 0.2kg |
| prepared into | 100,000 dripping pill |

Preparation process: the ginkgo terpene lactone was passed through a 60-mesh sieve, and other auxiliary materials were passed through a 100-mesh sieve. 1 kg of the ginkgo terpene lactone was mixed with 0.5 kg of silicified microcrystalline celluloseSMCC 50 and 0.1kg of methylcellulose in a tank mixer for 10 minutes to obtain a mixture. The mixture was crushed in a jet mill at an air pressure of 0.5 MPa to obtain a fine powder with d90 less than 20µm. The fine powder was mixed thoroughly with 0.2 kg of polyethylene glycol 400, so that they were fully contact with each other to obtain a second mixture. 3kg polyethylene glycol 4000, 0.1kg of hydroxypropyl methylcellulose K4 and 0.2kg of polysorbate-80 were heated to melt at 80°C, and then the second mixture was slowly added thereto under stirring to obtain a third mixture. The third mixture was dripped with a 2.4mm dripper, and then cooled and molded in liquid paraffin at 15°C to form 100,000 dropping pills, with each pill 51 mg.

### Example 6

Formulation:

| | |
|---|---|
| ginkgo terpene lactone | 1kg |
| mannitol | 1kg |
| polyethylene glycol 6000 | 3kg |
| lecithin | 0.2kg |
| prepared into | 100,000 dripping pills |

The mannitol used in formulation was prepared by spray drying.

Preparation process: the ginkgo terpene lactone was passed through a 60-mesh sieve, and other auxiliary materials were passed through a 100-mesh sieve. The ginkgo terpene lactone was crushed by a universal pulverizer to obtain a fine powder with d90 less than 50µm. 1kg of the fine powder of ginkgo terpene lactone was mixed thoroughly with 1kg mannitol, so that they were fully contact with each other to obtain a first mixture. 3kg polyethylene glycol 6000 and 0.2kg lecithin were heated to melt at 80°C, and then the first mixture was slowly added thereto under stirring to obtain a second mixture. The second mixture was dripped with a 2.4mm dripper, and then cooled and molded in liquid paraffin at 15°C to form 100,000 dropping pills, with each pill 52 mg.

### Example 7

Formulation:

| | |
|---|---|
| ginkgo terpene lactone | 1kg |
| microcrystalline celluloseUF711 | 0.8kg |
| calcium hydrogen phosphate | 0.2kg |
| polyethylene glycol 4000 | 2.8kg |
| polyethylene glycol 6000 | 0.4kg |
| prepared into | 100,000 dripping pills |

Preparation process: the ginkgo terpene lactone was passed through a 60-mesh sieve, and other auxiliary materials were passed through a 100-mesh sieve. The ginkgo terpene lactone, 0.8kg of microcrystalline cellulose UF711 and 0.2kg of calcium hydrogen phosphate were crushed by a universal pulverizer to obtain a fine powder with d90 less than 50µm. 2.8kg polyethylene glycol 4000 and 0.4kg polyethylene glycol 6000 were heated to melt at 80°C, and then the fine powder was added thereto slowly under stirring to obtain a mixture. The mixture was dripped with a 2.4mm dripper, and then cooled and molded in liquid paraffin at 15°C to form 100,000 dropping pills, with each pill 52 mg.

### Example 8

Formulation:

| | |
|---|---|
| ginkgo terpene lactone | 1kg |
| microcrystalline cellulosePH302 | 0.6kg |
| lactose | 0.4kg |
| polyethylene glycol 4000 | 2.8kg |
| prepared into | 100,000 dripping pills |

Preparation process: the ginkgo terpene lactone was passed through a 60-mesh sieve, and other auxiliary materials were passed through a 100-mesh sieve. 1kg of the ginkgo terpene lactone was mixed with 0.6kg of microcrystalline cellulose PH302 in a tank mixer for 10 minutes to obtain a mixture. The mixture was crushed in a jet mill at an air pressure of 0.5MPa to obtain a fine powder with d90 less than 20µm. The fine powder was mixed thoroughly with 0.4kg lactose, so that they were fully contact with each other to obtain a second mixture. 2.8kg polyethylene glycol 4000 was heated to melt at 80°C, the then second mixture was slowly added thereto under stirring to obtain a third mixture. The third mixture was dripped with a 2.4mm dripper, and then cooled and molded in liquid paraffin at 15°C to form 100,000 dropping pills, with each pill 48 mg.

### Example 9

Formulation:

| | |
|---|---|
| ginkgo terpene lactone | 1kg |
| microcrystalline cellulosePH302 | 0.4kg |
| lactose | 0.6kg |
| polyethylene glycol 4000 | 2.7kg |
| polysorbate-80 | 0.1kg |
| CMC-Na | 0.2kg |
| prepared into | 100,000 dripping pills |

Preparation process: the ginkgo terpene lactone was passed through a 60-mesh sieve, and other auxiliary materials were passed through a 100-mesh sieve. 1kg of the ginkgo terpene lactone was mixed with 0.4kg of microcrystalline cellulose PH302 in a tank mixer for 10 minutes to obtain a mixture. The mixture was crushed in a jet mill at an air pressure of 0.5MPa to obtain a fine powder with d90 less than 20µm. The fine powder was mixed thoroughly with 0.6kg lactose, so that they were fully contact with each other to obtain a second mixture. 2.7kg polyethylene glycol 4000 was heated to melt at 80°C, and then the second mixture together with 0.1kg polysorbate-80 and 0.2kg CMC-Na were slowly added thereto under stirring to obtain a third mixture. After fully stirring, the third mixture was dripped through a 2.4mm dripper, and then cooled and molded in liquid paraffin at 15°C to form 100,000 dropping pills, with each pill 50 mg.

### Comparative Example 1 (i.e. Example 4 of CN 106924197A)

Formulation:

| | |
|---|---|
| ginkgo terpene lactone | 1kg |
| polyethylene glycol 4000 | 1.75kg |
| polyethylene glycol 6000 | 1.75kg |
| polysorbate-80 | 0.1kg |
| CMC-Na | 0.5kg |
| prepared into | 100,000 dripping pills |

Preparation process: the ginkgo terpene lactone was passed through a 60-mesh sieve, and other auxiliary materials were passed through a 100-mesh sieve. 3kg of the polyethylene glycol 4000 and 1kg of polyethylene glycol 6000 were heated to melt at 80°C, and then a fine powder of ginkgo terpene lactone was slowly added thereto under stirring to obtain a mixture. The mixture was dripped with a 2.4mm dripper, and cooled and molded in liquid paraffin at 15°C to form 100,000 dropping pills, with each pill 51 mg.

Measurement of content: the content was measured by HPLC method according to "Pharmacopoeia 2010", and the results showed that contents of ginkgolide A, ginkgolide B, ginkgolide C and bilobalide in each pill are 2.5 mg, 1.0 mg, 1.5 mg, and 5.0 mg, respectively.

The beneficial effects of the present application are further illustrated with the following test examples.

### Test example 1

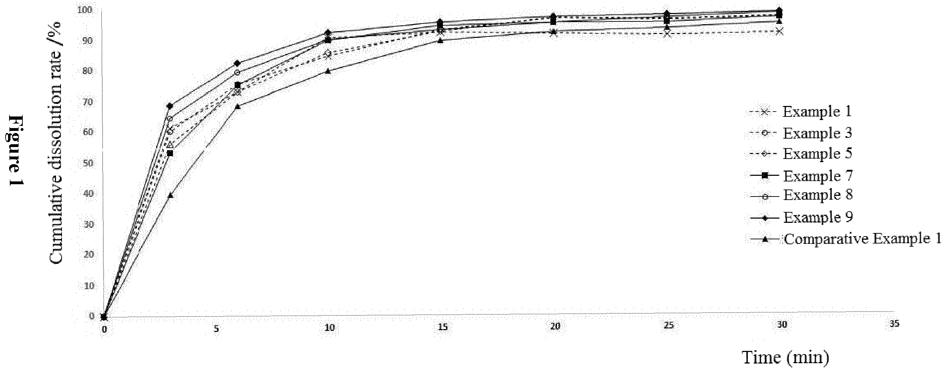

The ginkgo terpene lactone dropping pills prepared in the above examples 1, 3, 7, 9, and the comparative example 1 were selected to carry out in vitro dissolution test in a hydrochloric acid solution of pH 1.0 (pH in the stomach before meals). Samples were taken at 3 min, 6 min, 10 min, 15 min, 20 min, 25 min, and 30 min respectively to draw the dissolution curve, and the results are shown in Table 1 and Figure 1. The dissolution amount refers to a weight percentage of the total amount of ginkgolide A, ginkgolide B, ginkgolide C, ginkgolide M, ginkgolide J and bilobalide.

**Table 1: Dissolution test of the ginkgo terpene lactone dropping pills of the present application in hydrochloric acid solution of pH 1.0**

| Cumulative dissolution rate (%) | Time (min) | | | | | | |
|---|---|---|---|---|---|---|---|
| | 3 | 6 | 10 | 15 | 20 | 25 | 30 |
| Example 1 | 61.4 | 73.1 | 90.5 | 92.2 | 91.6 | 91.1 | 91.8 |
| Example 3 | 60.3 | 75.6 | 84.7 | 93.2 | 96.6 | 96.1 | 97 |
| Example 5 | 56.1 | 73.2 | 85.6 | 92.6 | 96.8 | 96.4 | 97.1 |
| Example 7 | 53.3 | 75.2 | 89.7 | 94.5 | 95.2 | 95.4 | 96.8 |
| Example 8 | 64.6 | 79.5 | 90.2 | 93.2 | 95.4 | 96.8 | 98.3 |
| Example 9 | 68.7 | 82.4 | 92.4 | 95.5 | 97.1 | 97.7 | 98.6 |
| Comparative Example 1 | 39.7 | 68.5 | 79.6 | 89.6 | 92.4 | 93.3 | 95.1 |

The above test results show that the ginkgo terpene lactone dropping pills prepared in examples of the present application have a faster dissolution rate than the ginkgo terpene lactone dropping pill prepared by the conventional formulation and method (Comparative Example 1, i.e, Example 4 of CN 106924197A) in the in vitro dissolution test. In the test, it is surprisingly found that the ginkgo terpene lactone dropping pills prepared in Example 8 and Example 9 using two kinds of fillers (microcrystalline cellulose and lactose) show significantly faster in vitro dissolution rate than the pills prepared in Example 1, Example 3, Example 5, and Example 7, showing unexpected technical effects.

### Test example 2

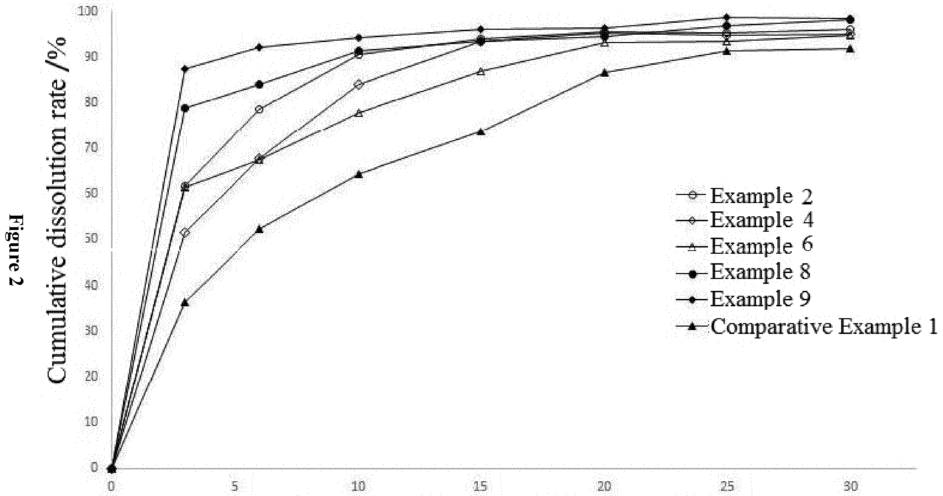

The ginkgo terpene lactone dropping pills prepared in the above examples 2, 4, 8, and 9, and the comparative example 1 were selected to carry out in vitro dissolution test in a phosphate buffer solution of pH 2.5 (i.e. pH in the stomach after meals). Samples were taken at 3 min, 6 min, 10 min, 15 min, 20 min, 25 min, and 30 min respectively to draw the dissolution curve, and the results are shown in Table 2 and Figure 2. The dissolution amount refers to a weight percentage of the total amount of ginkgolide A, ginkgolide B, ginkgolide C, ginkgolide M, ginkgolide J and bilobalide.

**Table 2: Dissolution test of ginkgo terpene lactone dropping pills of the present application in phosphate buffer solution of pH 2.5**

| Cumulative dissolution rate (%) | Time (min) | | | | | | |
|---|---|---|---|---|---|---|---|
| | 3 | 6 | 10 | 15 | 20 | 25 | 30 |
| Example 2 | 61.8 | 78.6 | 90.6 | 94.1 | 95.5 | 95.2 | 96 |
| Example 4 | 51.4 | 67.7 | 84.2 | 93.6 | 95.4 | 94.8 | 95.1 |
| Example 6 | 61.5 | 67.4 | 77.9 | 87.1 | 93.2 | 93.5 | 94.8 |
| Example 8 | 78.8 | 84.2 | 91.4 | 93.6 | 94.5 | 96.8 | 98.1 |
| Example 9 | 87.6 | 92.2 | 94.3 | 96.1 | 96.4 | 97.8 | 98.5 |
| Comparative Example 1 | 36.5 | 52.4 | 64.4 | 73.6 | 86.8 | 91.4 | 92 |

The above test results show that the ginkgo terpene lactone dropping pills prepared in examples of the present application have a faster dissolution rate than the ginkgo terpene lactone dropping pill prepared by the conventional formulation and method (Comparative Example 1, i.e, Example 4 of CN 106924197A) in the in vitro dissolution test. In the test, it is surprisingly found that the ginkgo terpene lactone dropping pills prepared in Example 8 and Example 9 using two kinds of fillers (microcrystalline cellulose and lactose) show significantly faster in vitro dissolution rates than the pills prepared in Example 2, Example 4 and Example 6, showing unexpected technical effects.

### Test example 3

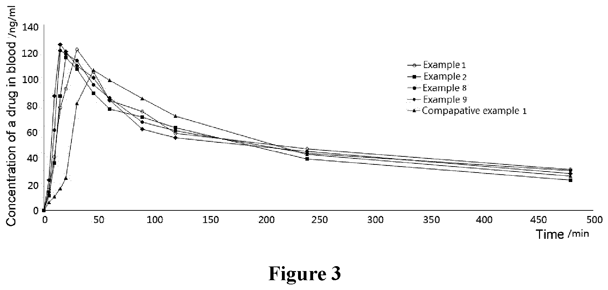

The ginkgo terpene lactone dropping pills prepared in Example 1 (using only one kind of filler having a rigid molecular structure, i.e. microcrystalline cellulose, as a filler), Example 2 (using only one kind of filler having a loose and porous surface, i.e. lactose, as a filler), Example 8 (using two kinds of fillers, i.e. microcrystalline cellulose and lactose), Example 9 (using microcrystalline cellulose, lactose, disintegration, and surfactant), and comparative example 1 (without filler) were administrated to rabbits by gavage at a dose of 500mg/kg. After the administration of the dropping pills, blood samples were collected directly into EDTA-Monovette blood collection tubes (Saaster Company) at 5min, 10min, 15min, 20min, 30min, 45min, 60min, 2h, 4h, and 8h, and centrifuged to prepare plasma samples. 30µL of plasma was added to 500µL tetrahydrofuran, 30µL I.S. solution (180 ng/mL) and 50µL 2M hydrochloric acid for each tube, and then mixed thoroughly to obtain a reaction mixture. The reaction mixture was vortexed for 20 seconds, and then centrifuged at 3700 g for 10 minutes to obtain a supernatant and a precipitate. 830µL of the supernatant was transferred to Extrelut^{®} extraction column, and then extraction was performed with 6 mL of methyl tert-butyl ether to obtain an extract, and the extract was dried by evaporation under nitrogen gas to obtain a residue. The residue was dissolved in a mixed solution of 50v/50v methanol/water (0.1% formic acid) and vortexed, then placed into an ultrasonic generator for treating for 15 minutes, and then centrifuged again at 3700 g for 10 minutes to obtain a supernatant which was transferred to a HPLC sample tube and analyzed. The concentration-time curve is drawn using the concentration of bilobalide (having the highest content) in blood. The results are shown in Table 3, Table 4 and Figure 3.

**Table 3: The drug concentrations of the ginkgo terpene lactone dropping pills of the present application in rabbits at various times**

| Drug concentration in blood (ng/ml) | Time (min) | | | | | |
|---|---|---|---|---|---|---|
| | 0 | 5 | 10 | 15 | 20 | 30 |
| Example 1 | 0 | 15.55 | 40.28 | 77.83 | 91.82 | 121.82 |
| Example 2 | 0 | 10.94 | 35.64 | 86.70 | 115.47 | 106.94 |
| Example 8 | 0 | 13.72 | 60.48 | 120.72 | 118.34 | 113.28 |
| Example 9 | 0 | 22.68 | 86.41 | 125.64 | 120.18 | 109.61 |
| Comparative Example 1 | 0 | 5.87 | 10.09 | 16.48 | 24.44 | 80.71 |

| Drug concentration in blood (ng/ml) | Time (min) | | | | | |
|---|---|---|---|---|---|---|
| | 45 | 60 | 90 | 120 | 240 | 480 |
| Example 1 | 104.87 | 83.23 | 74.78 | 58.28 | 46.54 | 30.87 |
| Example 2 | 88.53 | 76.70 | 70.38 | 62.39 | 38.70 | 22.90 |
| Example 8 | 95.07 | 84.94 | 66.74 | 60.21 | 43.18 | 29.96 |
| Example 9 | 100.32 | 83.59 | 61.28 | 54.83 | 44.66 | 27.75 |
| Comparative Example 1 | 106.18 | 98.41 | 84.65 | 71.33 | 42.15 | 25.69 |

**Table 4: Peak concentration and time to reach the peak concentration of ginkgo terpene lactone dropping pills of the present application in rabbits**

| Detection index | Example 1 | Example 2 | Example 8 | Example 9 | Comparative Example 1 |
|---|---|---|---|---|---|
| Tmax (min) | 30 | 20 | 15 | 15 | 45 |
| Cmax(ng/ml) | 121.82 | 115.47 | 120.72 | 125.64 | 106.18 |

Peak concentration (Cmax) and time (Tmax) to reach the peak concentration refer to the highest concentration of a drug in plasma after extravascular administration of the drug and time to reach the highest concentration of the drug, and represent the degree and speed of drug absorption, respectively.

The above test results show that compared with the ginkgo terpene lactone dropping pill prepared by the conventional formulation and method (Comparative Example 1, ie, Example 4 of CN 106924197A), the ginkgo terpene lactone dropping pills prepared in the examples of the present application reach the highest drug concentration in blood faster, the time Tmax to reach the peak are earlier, and the peak concentration Cmax value are higher in animal experiments. Therefore, ginkgo terpene lactones as the active components of the ginkgo terpene lactone dropping pills disclosed in the present application are easier to be absorbed and can exert the drug effect faster.

Compared with Example 1 and Example 2, the ginkgo terpene lactone dropping pills prepared in Examples 8 and 9 using two kinds of fillers (microcrystalline cellulose and lactose) have a significantly earlier Tmax value, showing unexpected technical effects. The Cmax value of Example 9 is significantly higher than that of Example 1, Example 2 and Example 8, and is the most preferred embodiment.

In summary, the present application provides a ginkgo terpene lactone dropping pill and a preparation method therefor. The dropping pill can be dissolved faster in vivo and in vitro and enter the blood faster, thus achieving therapeutic effects faster.

The references to the methods of treatment by therapy or surgery or *in vivo* diagnosis methods in **Test example 3** of this description are to be interpreted as references to compounds, pharmaceutical compositions and medicaments of the present invention for use in those methods.

## Claims

1. A dropping pill comprising:
i) an active component;
ii) a filler having a rigid molecular structure, and/or a filler having a loose and porous surface; and
iii) a matrix;
wherein the active component consists essentially of ginkgo terpene lactone,
the filler having a rigid molecular structure is selected from the group consisting of microcrystalline cellulose, methyl cellulose, ethyl cellulose, calcium hydrogen phosphate, calcium dihydrogen phosphate, calcium carbonate, calcium sulfate dihydrate, lactose, mannitol, cellulose lactose, and any combination thereof,
the filler having a loose and porous surface is selected from the group consisting of lactose, mannitol, cellulose lactose, any combination thereof, and
the dropping pill comprises 5-15 parts by weight of the ginkgo terpene lactone, 20-40 parts by weight of a matrix, and 1-20 parts by weight of the filler.

2. The dropping pill according to claim 1, **characterized in that**, the microcrystalline cellulose has a bulk density of 0.21 g/cm³ to 0.42 g/cm³.

3. The dropping pill according to claim 1, **characterized in that**, the dropping pill further comprises 0.1-4 parts by weight of a surfactant selected from the group consisting of polysorbate-80, lecithin and carbomer.

4. The dropping pill according to claim 3, **characterized in that**, the dropping pill further comprises 0.1-2.5 parts by weight of a disintegrant selected from the group consisting of sodium carboxymethyl cellulose, dry starch, low-substituted hydroxypropyl cellulose, cross-linked polyvinylpyrrolidone and cross-linked sodium carboxymethyl cellulose.

5. The dropping pill according to claim 4, **characterized in that**, the filler is a mixture of microcrystalline cellulose and lactose.

6. The dropping pill according to claim 5, **characterized in that**, a weight ratio of the microcrystalline cellulose to lactose is (4-1): (1-4).

7. A method for preparing the dropping pill according to any one of claims 1 to 6, **characterized in that** the method comprises the following steps of:
a) co-crushing or mixing the ginkgo terpene lactone and the filler to obtain a fine powder;
b) mixing a matrix with other components and heating them to melt, adding the fine powder obtained in step a) thereto under stirring to obtain a mixture; and
c) dripping the mixture obtained in step b) with a dripper, and cooling with a condensate to form a dripping pill.

8. The method according to claim 7, **characterized in that**, the crushing in step a) is crushing by gas impact and/or crushing by mechanical shear.

9. The method according to claim 8, **characterized in that**, the crushing by gas impact is performed at a pressure of 0.5-0.8 MPa.

## Patentansprüche

1. Tropftablette umfassend:
i) einen Wirkstoff;
ii) einen Füllstoff, der eine starre Molekularstruktur aufweist und/oder ein Füllstoff, der eine lockere und poröse Oberfläche aufweist; und
iii) eine Matrix;
wobei der Wirkstoff im Wesentlichen aus Ginkgo-Terpenlacton besteht,
wobei der Füllstoff, der eine starre Molekularstruktur aufweist, ausgewählt ist aus der Gruppe bestehend aus mikrokristalliner Cellulose, Methylcellulose, Ethylcellulose, Calciumhydrogenphosphat, Calciumdihydrogenphosphat, Calciumcarbonat, Calciumsulfatdihydrat, Lactose, Mannitol, Celluloselactose und jeder beliebigen Kombination davon,
wobei der Füllstoff, der eine lockere und poröse Oberfläche aufweist, ausgewählt ist aus der Gruppe bestehend aus Laktose, Mannitol, Celluloselaktose, jeder beliebigen Kombination davon und
die Tropftablette zu 5-15 Gewichtsteilen Ginkgo-Terpenlacton, zu 20-40 Gewichtsteilen eine Matrix und zu 1-20 Gewichtsteilen den Füllstoff umfasst.

2. Tropftablette nach Anspruch 1, **dadurch gekennzeichnet, dass** die mikrokristalline Cellulose eine Schüttdichte von 0,21 g/cm³ bis 0,42 g/cm³ aufweist.

3. Tropftablette nach Anspruch 1, **dadurch gekennzeichnet, dass** die Tropftablette ferner zu 0,1-4 Gewichtsteilen ein Tensid, ausgewählt aus der Gruppe bestehend aus Polysorbat-80, Lecithin und Carbomer, umfasst.

4. Tropftablette nach Anspruch 3, **dadurch gekennzeichnet, dass** die Tropftablette ferner zu 0,1-2,5 Gewichtsteilen ein Sprengmittel, ausgewählt aus der Gruppe bestehend aus Natriumcarboxymethylcellulose, trockener Stärke, niedrigsubstituierter Hydroxypropylcellulose, vernetztem Polyvinylpyrrolidon und vernetzter Natriumcarboxymethylcellulose, umfasst.

5. Tropftablette nach Anspruch 4, **dadurch gekennzeichnet, dass** der Füllstoff eine Mischung aus mikrokristalliner Cellulose und Laktose ist.

6. Tropftablette nach Anspruch 5, **dadurch gekennzeichnet, dass** ein Gewichtsverhältnis der mikrokristallinen Cellulose zu Laktose (4-1) : (1-4) beträgt.

7. Verfahren zum Herstellen der Tropftablette nach einem der Ansprüche 1 bis 6, **dadurch gekennzeichnet, dass** das Verfahren die folgenden Schritte umfasst:
a) gemeinsames Zerkleinern oder Mischen des Ginkgo-Terpenlactons mit dem Füllstoff, um ein feines Pulver zu erhalten;
b) Mischen einer Matrix mit anderen Komponenten und Erhitzen dieser zum Schmelzen, dazu Hinzufügen des in Schritt a) erhaltenen feinen Pulvers unter Rühren, um eine Mischung zu erhalten; und
c) Tropfen der in Schritt b) erhaltenen Mischung mit einem Tropfer und Abkühlen mit einem Kondensat, um eine Tropftablette auszubilden.

8. Verfahren nach Anspruch 7, **dadurch gekennzeichnet, dass** das Zerkleinern in Schritt a) ein Zerkleinern durch Gasaufprall und/oder Zerkleinern durch mechanische Scherung ist.

9. Verfahren nach Anspruch 8, **dadurch gekennzeichnet, dass** das Zerkleinern durch Gasaufprall bei einem Druck von 0,5-0,8 Mpa durchgeführt wird.

## Revendications

1. Pilule obtenue par égouttement comprenant :
i) un composant actif ;
ii) une charge ayant une structure moléculaire rigide, et/ou une charge ayant une surface libre et poreuse ; et
iii) une matrice ;
dans laquelle le composant actif consiste essentiellement en lactone terpénique de ginkgo,
la charge ayant une structure moléculaire rigide est choisie dans le groupe constitué de cellulose microcristalline, méthylcellulose, éthylcellulose, hydrogénophosphate de calcium, dihydrogénophosphate de calcium, carbonate de calcium, sulfate de calcium dihydraté, lactose, mannitol, cellulose-lactose, et toute combinaison de ceux-ci,
la charge ayant une surface libre et poreuse est choisie dans le groupe constitué de lactose, mannitol, cellulose-lactose, toute combinaison de ceux-ci, et
la pilule obtenue par égouttement comprend 5 à 15 parties en poids de la lactone terpénique de ginkgo, 20 à 40 parties en poids d'une matrice, et 1 à 20 parties en poids de la charge.

2. Pilule obtenue par égouttement selon la revendication 1,
**caractérisée en ce que** la cellulose microcristalline a une masse volumique apparente de 0,21 g/cm³ à 0,42 g/cm³.

3. Pilule obtenue par égouttement selon la revendication 1,
**caractérisée en ce que** la pilule obtenue par égouttement comprend en outre 0,1 à 4 parties en poids d'un agent tensioactif choisi dans le groupe constitué de polysorbate-80, lécithine et carbomère.

4. Pilule obtenue par égouttement selon la revendication 3,
**caractérisée en ce que** la pilule obtenue par égouttement comprend en outre 0,1 à 2,5 parties en poids d'un désintégrant choisi dans le groupe constitué de carboxyméthylcellulose sodique, amidon sec, hydroxypropylcellulose faiblement substituée, polyvinylpyrrolidone réticulée et carboxyméthylcellulose sodique réticulée.

5. Pilule obtenue par égouttement selon la revendication 4,
**caractérisée en ce que** la charge est un mélange de cellulose microcristalline et de lactose.

6. Pilule obtenue par égouttement selon la revendication 5,
**caractérisée en ce qu'un** rapport en poids entre la cellulose microcristalline et le lactose est de (4-1): (1-4).

7. Procédé de préparation d'une pilule obtenue par égouttement selon l'une quelconque des revendications 1 à 6, **caractérisé en ce que** le procédé comprend les étapes suivantes consistant à :
a) broyer ensemble ou mélanger la lactone terpénique de ginkgo et la charge afin d'obtenir une poudre fine ;
b) mélanger une matrice avec d'autres composants et les chauffer afin de les faire fondre, en y ajoutant la poudre fine obtenue à l'étape a) sous agitation pour obtenir un mélange ; et
c) effectuer un égouttement du mélange obtenu à l'étape b) à l'aide d'un dispositif d'égouttement et le refroidir avec un condensat afin de former une pilule obtenue par égouttement.

8. Procédé selon la revendication 7, **caractérisé en ce que** le broyage à l'étape a) est un broyage par impact de gaz et/ou un broyage par cisaillement mécanique.

9. Procédé selon la revendication 8, **caractérisé en ce que** le broyage par impact de gaz est effectué à une pression de 0,5 à 0,8 MPa.
